# EUROPÄISCHE PATENTSCHRIFT

(11) **EP 2 842 550 B1**
(45) Veröffentlichungstag und Bekanntmachung des Hinweises auf die Patenterteilung: **22.02.2017**
(21) Anmeldenummer: 14180685.1
(22) Anmeldetag: 12.08.2014
(51) Int. Cl.: A61K 9/48, A61K 9/50, A61K 9/16, A61K 31/685, A61K 31/198, A61K 31/57, A61K 31/194

(54) **Schwammkollagen umfassende Zubereitungen mit definiertem in vivo-Freisetzungsprofil vor allem im Colon, deren Herstellung und Verwendung**
Compositions comprising sponge collagen with a defined in vivo release profile, especially in the colon, their preparation and use
Compositions comprenant de collagen d'eponge avec un in vivo profil defini de libearation de preference dans le colon, leurs preparation et utilisation

(30) Priorität: 29.08.2013 DE 102013014417
(43) Veröffentlichungstag der Anmeldung: 04.03.2015
(73) Patentinhaber: KliniPharm GmbH, 60313 Frankfurt/Main (DE)
(72) Erfinder: Schatton, Dr., Wolfgang, 65760 Eschborn (DE)
(74) Vertreter: Müller, Claudia

(56) Entgegenhaltungen:
- DE-A1- 10 010 113
- DE-A1- 19 857 750
- PERGAMENT V; SCHATTON W; FOTAKI N: "Study of release properties from marine sponge collagen coated formulations", PROC AAPS, Bd. 151, 2011, Seite R6121, XP002734574,
- NICKLAS M; SCHATTON W; HEINEMANN S; HANKE T; KREUTER J.: "Enteric coating derived from marine sponge collagen", DRUG DEV IND PHARM., Bd. 35, Nr. 11, 2009, Seiten 1384-8, XP002734575,
- DATABASE WPI Week 200866 Thomson Scientific, London, GB; AN 2008-L16224 XP002734576, & CN 101 130 066 A (LI L) 27. Februar 2008 (2008-02-27)

## Beschreibung

### Gegenstand der Erfindung

Die vorliegende Erfindung betrifft Zubereitungen oder Arzneiformen, vor allem enterale, vorzugsweise orale Zubereitungen, mit definiertem in vivo-Freisetzungsprofil vor allem im Colon eines Säugetiers, insbesondere des Menschen. Diese Zubereitungen umfassen feste bis pastenförmige Matrizes und weisen Talkum-freies Schwammkollagen als vorzugsweise externes oder integriertes Überzugsmittel in vor allem geringen Mengen wie vor allem weniger als 5% auf. Derartige Produkte können auf einfache Weise hergestellt werden. Die Matrizes können dabei beliebig ausgewählt werden aus herkömmlichen, für die orale Verabreichung bekannten Darreichungsformen wie Tabletten, Dragees, Pasten, Granulaten, Kapseln, auch als Mikro-/Nano-Kapseln, Suspensionen. Diese Matrizes weisen selbst Kollagen bzw. Kollagenfasern oder Kollagenpartikel aus Schwämmen auf, nämlich marines denaturiertes Schwammkollagen als Träger oder Teil der Trägermasse Dieses Matrixkollagen bzw. die Matrix ist insbesondere wenigstens zum Teil oder vollständig strukturell verschieden vom Überzugskollagen. Als Schwämme zur Gewinnung des Überzugsmaterials eignen sich insbesondere Meeresschwämme, vor allem solche aus der Klasse der Demospongiae und deren 11 Ordnungen, z.B. den Geodiidae, Chondrosiidae, Suberitidae, Axinellidae, Halichondridae, Dysideidae, Songiidae und andere.

Die Erfindung betrifft auch die Herstellung und Verwendung derartiger Zubereitungen für die in vivo-Anwendung zur definierten Freisetzung von Wirkstoffen, vor allem im Colon sowie die Verwendung von Schwammkollagen als Colon-spezifisches Retardierungsmittel für arznellich/pharmakologisch wirksame Substanzen (Wirkstoffe). Schwammkollagen kann somit erfindungsgemäß eingesetzt werden zur Herstellung von Zubereitungen für eine kontrollierte Wirkstofffreisetzung im Colon. Überraschender Weise zeigte sich, dass der oder die Wirkstoffe anders als bisher aus den in-vitro Daten anzunehmen war, während der Darmpassage nicht kontinuierlich, sondern im Colon punktuell-quantitativ freigesetzt werden. Dies führt zu einer erheblichen Reduktion der erforderlichen Wirkstoffmenge. Dadurch können auch Nebenwirkungen, welche hier bei der klassischen oralen Gabe auftreten, vermieden werden. Als Wirkstoffe sind insbesondere Säuregruppen-haltige Substanzen geeignet, obgleich diese ein hohes Auflösungsvermögen im Magenbereich aufweisen können. Dazu gehören z.B. Aminosäuren, Eiweißstoffe, aber auch Wachstumsfaktoren, lipophile sowie hydrophile, positiv, neutral oder negativ geladene Aktivstoffe. Ebenfalls können zahlreiche Xenobiotica in die erfindungsgemäßen Zubereitungen hineingearbeitet werden, besonders solche, die nach oraler Gabe infolge eines First-Pass Effektes bereits metabolisiert werden, wie z.B. auch Hormone. Vor allem geeignet sind die erfindungsgemäßen Zubereitungen für das Colon-Targeting von Wirkstoffen, z.B. für die Behandlung ZNS-abhängiger neurodegenerativer Erkrankungen, ausgewählt aus Lecithinen wie Ei-Lecithin, Phospholipiden, Hormonen wie Progesteron, Antioxidantien, wie z.B. Coenzym Q10, Grünteeextrakte u.a. oder kurzkettigen Fettsäuren, wie Natriumbutyrat bzw. Fumarsäure und deren Derivaten, bei Colitis Ulcerosa bzw. metastierendem Coloncarcinomen, Mitteln zur Rekonstitution der Darmschleimhaut, Antibiotika und Symbionten zur Entkeimung und Beeinflussung der Darmflora, oder Fumarsäure bei MS oder bei Psoriasis, desgleichen Progesteron und anderen Hormonen, orthomolekularen Wirkstoffen, wie Orotsäure, weiteren Aminosäuren und ZNS-aktiven Pflanzenextrakten. Die Zubereitungen oder Arzneiformen können auch als Suspensionen in Hydro- oder Lipo-Gelen, Pasten, Schäume, letztere auch getrocknet, vorliegen. Diese können dann in geeigneten Verabreichungsformen wie Kapseln gefüllt und sodann erfindungsgemäß überzogen werden. Sie lassen sich bevorzugt auf Schleimhäute des Magen-Darmtrakts verbringen und können so nach Lösung der Umhüllung z. B. bei geeigneten Träger- und Wirkstoffen wie Kollagen, insbesondere solches aus (Meeres)-Schwämmen, als Gewebeersatz, oder zum Abdecken, Abschirmen oder zur Rekonstitution physiologischer Oberflächen des Gastrointestinaltrakts, insbesondere des Colons, oder als Barrieren gegen pathogene Keime z.B. bei Sepsis, bei HIV-Infektionen, oder bei Neurodermitis, allergischem Asthma und zur Förderung der Wundheilung eingesetzt werden, da sie direkt am genannten Wirkort freigesetzt werden.

### Stand der Technik

Zubereitungen zur Freisetzung von Wirkstoffen sind hinreichend bekannt. Dabei kann es erforderlich sein, dass diese sofort oder erst nach einer gewissen Zeit oder kontinuierlich freigesetzt werden. Dies hängt vom jeweiligen Wirkstoff und der Indikation ab. Seit langem wird daher versucht, die Wirkstofffreisetzung spezifisch zu steuern. Eine Steuerung ist beispielsweise über die Teilchengröße/Teilchenart (d. h. über physikalisch-chemische Methoden bei der Herstellung der Wirkstoffmatrizes) oder durch Umhüllungstechniken möglich, siehe DD 00295538 A5. So kann die Freisetzung aus Granulaten z. B. sofort und aus Pellets und/oder spezifisch mit Filmen umhüllten Matrizes verzögert erfolgen. Eine derartige Freisetzungsvarianz ist z. B. bei Schmerzmitteln wichtig, siehe DE 10 2005 054610 B4. Andere Wirkstoffe können z.B. sowohl akut als auch retardiert erforderlich werden, siehe DE 000069735581 T2.

Dies entspricht bis dato bekannten Methoden zur Freisetzung von Wirkstoffen. Zur retardierten Freisetzung, insbesondere in der Nähe des Colons, werden häufig sehr aufwendige Zeit-, pH-Wert- oder Mikroflora-kontrollierte Verfahren unter Einsatz spezieller Retardierungsmittel, insbesondere als Überzug angewendet oder Kombinationen dieser Verfahren. Am bekanntesten sind die zahlreichen, mit Poly-(meth-)acrylat-Derivaten der Fa. Evonik, Darmstadt, z.B. Eudragit® L oder S100, überzogenen Systeme. So beschreibt die DE 19857750 A1 die Herstellung von oral verabreichbarem Phosphatidylcholin, das mit Acrylpolymeren überzogen wurde, wie z. B. einer Kombination aus Eugradit® S 100 und L-Verbindungen, die eine verzögerte Freisetzung bei pH > 6,4, im terminalen Ileum ermöglichen soll.

Bei Untersuchungen mit freiwilligen Probanden wurden für Eudragit® überzogene Colon-Targeting-Systeme allerdings variable Freisetzungsorte, vom Ileum bis zum distalen Colon reichend, sowie sehr unterschiedliche Freisetzungszeiten nachgewiesen. Teilweise blieb die Freisetzung völlig aus (Ashford et al., An in-vivo investigation into the suitability of pH dependent polymers for colonic targeting. Int. J. Pharm. 95, 193 ff, 1993). Dies zeigt, dass der gastrointestinale pH-Wert kein ausreichend stabiler Parameter für die gezielte Wirkstofffreisetzung im Dickdarm ist, da er stark von Ernährungsgewohnheiten, der Darmmotilität und anderen inter- oder intraindividuellen Schwankungen beeinflusst wird.

Auch muss man hohe Konzentrationen, im Falle des Coatings des Beispiels DE 19857750 A1 bis zu 60% und mehr an z.B. Eudragit S100 als Hilfsstoff einsetzen, um pharmazeutische Wirkstoffe in die Nähe des Colonbereichs des Darms transportieren und dort freisetzen zu können.

Eine Überlegung zur Lösung der oben beschriebenen Probleme führte in jüngster Zeit dazu, Schwammkollagen im Hinblick auf eventuelle Coating-Eigenschaften zu testen. Schwämme existieren beinahe unverändert seit mehr als 600 Millionen Jahren und gehören damit zu den ältesten Mitgliedern der Gruppe der Metazoa. Ihre zu Zellkolonien gruppierten einzelnen Zellen sind mit dem Bindegewebsprotein Kollagen verbunden. Schwammkollagen ist im Milieu des Meeres ein wasserunlösliches Protein, welches das klassische Aminosäuremuster der bekannten tierischen Kollagene besitzt. Jede dritte Aminosäure ist z.B. Glycin und der Gehalt an Prolin sowie Hydroxyprolin ist typischerweise sehr hoch. Schwammkollagen zeichnet sich durch anti-oxidative Eigenschaften aus. Es ist je nach Verarbeitung im sauren oder im basischen bis stark basischen Medium gut löslich. B. Diehl und Seufert et al. beschreiben in J.Cell Sc. 79, 271-85 (1985) die Isolierung von Kollagen aus dem Schwamm Geodia cydonium und Chondrosia renifornis mit natürlicher Aminosäurezusammensetzung unter Vermeidung von Denaturierungsreagentien. Dies erfolgt durch Suspendierung von homogenisiertem frischen Material in Tris-HCl Puffer, pH-Wert Einstellung auf 9, Zentrifugation und Homogenisierung. Die Reinigung erfolgt durch Gelfiltration, wobei aber eine geringe Ausbeute (1,7 bzw. 3,1%) Kollagen erhalten wird. Ein verbessertes Verfahren ist aus der EP 1259 120 B1 bekannt. Dort wird die Herstellung von Schwammkollagen aus Meeresschwämmen beschrieben, indem diese in Alkohol, vorzugsweise Ethanol, aufgeschlämmt und sodann extrahiert und aufgearbeitet werden. Die Extraktion kann mit einem Puffer wie Tris-HCl-Puffer erfolgen. Eine enzymatische Behandlung, oder eine Gelfiltration ist hier nicht erforderlich. Granulate oder Nanopartikel aus derartig hergestelltem Schwammkollagen weisen entzündungshemmende Eigenschaften auf und können insbesondere als topische Mittel eingesetzt werden. Auch sind orale Verabreichungsformen, z. B. mit anderen Wirkstoffen möglich.

Wie eingangs erwähnt, wurde bereits versucht, ein Arzneimittelcoating mit Schwammkollagen durchzuführen. So berichten Nicklas et al. 2009 erstmals von in vitro- Versuchen mit Talkum-haltigem Schwammkollagen als filmartiges Überzugsmaterial. Es wurde festgestellt, dass mit 12,9 mg Schwammkollagen pro cm² Tablette ein reproduzierbarer Grad an Magensaftresistenz erreicht werden kann, der den Anforderungen des Europäischen Arzneibuchs entspricht, vgl. Nicklas M, Schatton W, Heinemann S, Hanke T, Kreuter J., Drug Dev Ind Pharm. 2009; 35(11):1384-8. Es handelte sich dabei um eine wirkstofffreie Tablette, d.h. ob ein Wirkstoff daraus ebenso oder anders freigesetzt ist, kann hieraus nicht abgeleitet werden. Das Experiment deutet jedoch darauf hin, dass ein Überzugsfilm aus Schwammkollagen eine Magensaftresistenz bewirken kann.

2 Jahre später untersuchten Pergament et al. die Freisetzung von 50 mg Diclofenac aus Granulaten und Kapseln auf Calciumchlorid-Basis, die mit 0,5 bis 5% (w/w) eines nicht näher charakterisierten Schwammkollagens Magensaft-resistent überzogen waren, in vitro in künstlichem Magensaft und künstlichem Dünndarm-Milieu im Vergleich zu einem magensaftresistenten 50 mg Diclofenac Handelspräparat mit einem Eudragit® (Acrylat)-Mehrfach-Überzug, ca. 60%, bezogen auf das Gesamtgewicht der Zubereitung) in einer Erweka^{®} USP IV Apparatur. Alle Formulierungen waren jeweils im synthetischen sauren Milieu stabil. Im synthetischen Darmmilieu wurde dann erwartungsgemäß Diclofenac freigesetzt, und zwar bei Eintreten des Darmmilieus (Dünndarm) (Pergament V, Schatton W, Fotaki N. Study of release properties from marine sponge collagen coated formulations. Proc AAPS. 2011; 151:R6121). Dabei zeigte sich, dass im Gegensatz zum im frühen Darm Ad-Hoc-Freisetzungsprofil der klassischen Magensaft resistenten Diclofenac-Formulierung mit Eudragit® ((Voltaren ® retard) die Freisetzungsraten aus den mit Schwammkollagen überzogenen Formulierungen eher linear kontinuierlich waren und in etwa einer Kinetik nullter Ordnung folgten. Schwammkollagen bewirkt somit nach diesen in vitro-Testen eine verzögerte kontinuierliche Wirkstofffreisetzung.

Das Problem der verzögerten Wirkstofffreisetzung ist auch in der CN101130066 (A) angesprochen. Laut Abstract wird eine Colon Targeting spezifische Zubereitung mit Tierhautkollagen - Polypeptiden in der Matrix beschrieben. Das Tierhautkollagen wird zusammen mit den Wirkstoffen in Mikro-Pellets gefüllt. Die Pellets werden sodann mit üblichen, magensaftresistenten Mitteln (Acrylaten wie Eudragit® S 100, Hydroxypropylmethylcellulose, Opadry) in größeren Mengen bis zu 62% gecoated. Dadurch soll eine Wirkstofffreisetzung im Darm erzielt werden. Welches Freisetzungsprofil hier vorliegt oder erreicht wird, ist nicht erläutert.

Bei tierischen Kollagenen, welche aus Tierhäuten sowie den Sehnen, Knorpeln und Knochen von Schweinen, Kälbern, Rind oder Fischen gewonnen werden, besteht jedoch die Gefahr des Einbringens von pathogen Keimen (z.B. BSE (boviner spongiformer Enzephalopathie). Dieses Problem wird mit der obigen Methode nicht gelöst. Insbesondere sind auch hier höhere Mengen an Überzugsmitteln mit bis zu 62 % ähnlich wie im klassischen Mittel Voltaren® nötig, um überhaupt eine gastro-stabile, intestinal-retardierte Formulierung zu erhalten, denn tierisches Kollagen löst sich im stark sauren Milieu und ist bei physiologischen bis stark basischen pH-Werten unlöslich und damit für Colon-Targeting nicht geeignet.

### Aufgabe der Erfindung

Der Erfindung liegt die Aufgabe zugrunde, eine Zubereitung zur Verfügung zu stellen, mit der in vivo ein definiertes Freisetzungsprofil eines Wirkstoffs im Darm, insbesondere im Dickdarm erreicht werden kann, ohne dass mehrfache Dickschichten herkömmlicher Überzüge z. B. aus Acrylaten, Cellulosederivaten oder Mischungen hiervon in Mengen bis zu 60 Gew.% und mehr, bezogen auf das Gewicht der Zubereitung, eingesetzt werden müssten. Ferner sollen gesundheitliche Risiken, welche durch Wirk- und Inhaltsstoffe selbst bedingt sind wie tierisches Ausgangsmaterial, vermieden werden. Schließlich wäre es von Vorteil, ein einfaches Verfahren zur Herstellung derartiger Zubereitungen zur Verfügung zu stellen, mit denen Wirkstoffe definiert im Colon freigesetzt werden können. Besonders erstrebenswert wäre es, durch eine gezielte Freisetzung eine Änderung, insbesondere Reduktion der bisher erforderlichen Wirkstoffmenge zu erreichen.

### Lösung der Aufgabe

Diese Aufgabe wird erfindungsgemäß gelöst durch die Bereitstellung von in vivo-Zubereitungen mit kontrolliertem Freisetzungsprofil insbesondere im Dickdarm, aus einer festen bis pastenförmigen Grundmatrix Wirkstoffformulierung), mit einem oder mehreren Trägerstoffen, ggf. Zusatzstoffen hierfür, sowie einem oder mehreren Wirkstoffen, welche als externen oder integrierten Überzug Schwammkollagen aufweist, insbesondere in geringen Mengen von bis zu 5 Gew.%, bezogen auf die Gesamtmenge der Zubereitung. Dabei ist das Überzugsmittel eine Lösung oder Suspension eines (vor allem marinen) Schwammkollagens in Wasser oder Wasser-Ethanol, die einen pH- Wert von ca. 6,0 bis 9 aufweist. Dazu können vorzugsweise pH-Regulatoren wie Säuren, Basen, Puffer vorhanden sein, insbesondere Phosphatpuffer, Ammoniumpuffer, sowie ggf. Konservierungsmittel, und/oder Viskositätsregulatoren. Talkum ist nicht im Überzugsmittel enthalten. Die (Grund-) -Matrix (Wirkstoffformulierung) kann dabei eine Tablette, ein Pulver, Mikropulver, Pellets, eine Paste, z. B. eine gelförmige Paste, Suspension oder ein Dragee, Granulat sein, wobei sich der Überzug über der bzw. um die Wirkstoffformulierung wie darin integriert befindet und zumindest teilweise vom Träger verschieden ist.

Überzug im Sinne vorliegender Erfindung bedeutet, dass das eingesetzte Schwammkollagen wie oben beschrieben extern um die Matrix appliziert sein kann wie dies z. B. durch Sprühen erfolgt oder in der Matrix integriert sein kann. Letzteres kann z. B. durch Mischen des Überzugsmittels wie beschrieben mit den Matrixbestandteilen und anschließendes Weiterverarbeiten wie Trocknen/Pressen, u.ä. erfolgen. Im Falle der Applikation um die Wirkstoffformulierung kann die Matrix das Überzugsmittel im Träger zu einem Teil umfassen (integriert sein). Falls die Matrix aus Kollagen besteht, so unterscheidet sich dieses strukturell vom Überzugskollagen. Insbesondere ist das Überzugskollagen nativ und das Matrixkollagen denaturiert.

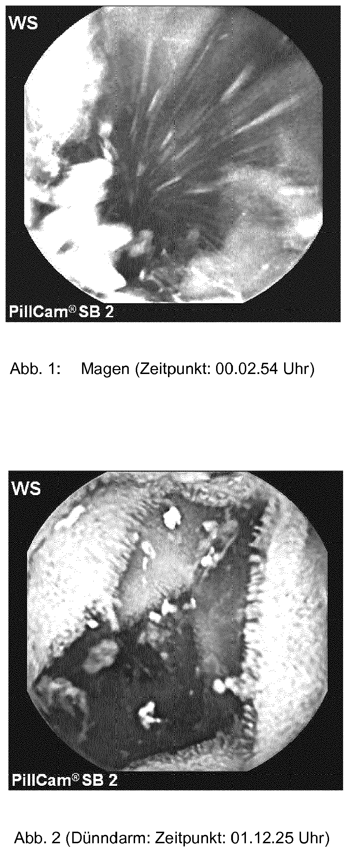

Erfindungsgemäß wird der externe Überzug üblicherweise in einer Schicht angewendet. Die Wirkstoffmatrix umfasst demnach neben dem/den Wirkstoffen einen oder mehrere Trägerstoffe, ggf. zusammen mit anderen Zusatzstoffen. Die feste oder pasten- bzw. gelförmige Matrix kann z. B. in Kapseln (Hart-/Weichgelatinekapseln), Mikrokapseln eingefüllt sein oder direkt als Pulver, Granulat, gepresste Tablette, Filmtablette oder Dragee, Paste verwendet werden. Somit wird die Matrix oder die diese enthaltende Verabreichungsform erfindungsgemäß mit einer wässrigen Lösung (oder) /Suspension (oder) /Dispersion oder Kombinationen hiervon von Kollagen wie oben beschrieben, hergestellt aus Meeresschwämmen, insbesondere erhalten durch Ethanolbehandlung und Extraktion mit wässrigen Systemen gemäß der EP 1259120 B1, z. B. durch Sprühung überzogen oder durch Mischen mit dem Überzug integriert versehen. Wie nachfolgendes Beispiel 4 sowie die Abbildung Nr.1 "Magen" zeigen, sind derartige Formulierungen magensaftresistent, wie dies bereits in den oben beschriebenen in vitro- Experimenten mit und ohne Wirkstoffe aufgezeigt wurde. Werden sie jedoch wie erfindungsgemäß hergestellt und oral in vivo verabreicht, ändert sich jedoch das Freisetzungsprofil im Gegensatz zu dem im in vitro-Experiment beschriebenen Muster überraschender Weise dahingehend, dass keine kontinuierliche Freisetzung mehr eintritt. Vielmehr bleibt die Zubereitung auch über den gesamten Dünndarmbereich stabil und wird erst im Dickdarm (Colon) in kurzer Zeit nahezu vollständig freigesetzt. Dabei wurde völlig überraschend gefunden, dass z.B. ein mit 2% der erfindungsgemäßen Schwammkollagendispersion/Lösung überzogenes Granulat - anders als es aus den linearen in-vitro Freisetzungsdaten zu erwarten war - erst im terminalen Ileum deutlich erkennbar in Kollagenfasern und seine weiteren Bestandteile zu zerfallen beginnt.

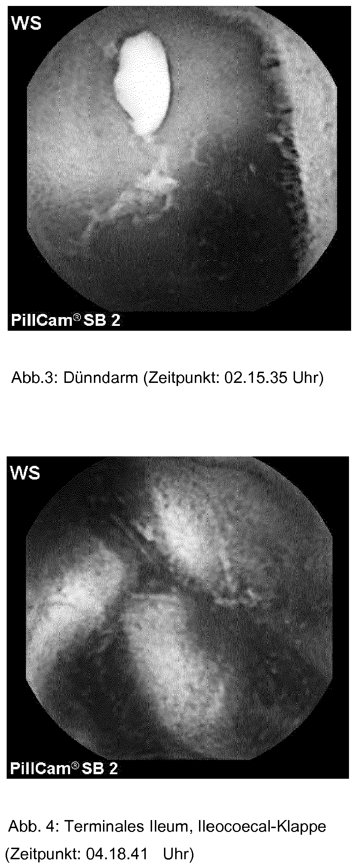

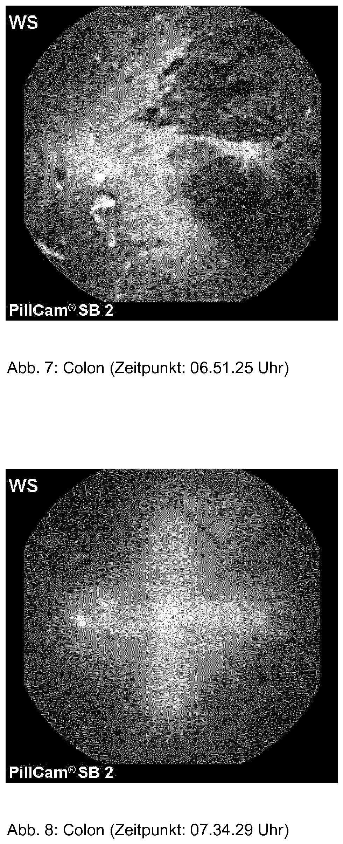

Wie nämlich die Abbildungen 1-8 gemäß Beispiel 4 veranschaulichen, quellen bei der Passage durch den Magendarmtrakt zunächst die Kollagenfasern des Überzugs erst im terminalen Ileum deutlich auf und sind plötzlich als einzelne Faserbündel auf den Aufnahmen erkennbar. Ohne an eine Theorie gebunden zu sein, wird vermutet, dass diese Fasern dann ein Gel bilden, das sich anschließend wie ein Film auf die Colon-Schleimhaut legt und diese vollständig abdeckt. Im Laufe der Colon-Passage erst werden die Schwammkollagenfasern ganz allmählich abgebaut und verlieren langsam und kontinuierlich an Faserlänge (vergl. die Abbildungen 1-3 (Magen, Dünndarm), terminales Ileum und Colon (Abb. 4-8).

Die lokal vorhandene höhere Wirkstoffdosis pro /Zeiteinheit führt zu einer verbesserten Behandlung, wobei ferner auch Nebenwirkungen eingeschränkt werden können. Erfindungsgemäß ist es dadurch auch möglich, die Wirkstoffmenge zu reduzieren, weil keine Transportverluste entstehen. Insbesondere sind auch weitaus geringere Mengen an Überzugsmitteln als bisher nötig, wobei es sich dabei zusätzlich um ein natürliches Material ohne wietere Synthetik handelt. Beispielsweise reichten bei den erfindungsgemäßen Formulierungen bereits ein Anteil kleiner 2% an Schwammkollagenen in der Ummantelung des Produktes, um eine gewünschte Menge an Substanzen im Colon freizusetzen. Wie aus Beispiel 4 hervorgeht, konnte die Wirkstoffmenge/Wirkstoffart erheblich geändert werden: so wurde anstatt Fumarsäuredimethylester, bekannt z.B. als Fumaderm® (neu Tecfidera ®) zur Behandlung ZNS-abhängiger Erkrankungen (Multiple Sklerose) anstatt der bisher verabreichten Dosis von 250 mg lediglich 30 mg der sehr viel besser verträglichen freien physiologischen Fumarsäure bei sehr erfolgreicher Therapie eingesetzt. Ein derartiges Ergebnis war im Hinblick auf den Stand der Technik nicht zu erwarten.

Somit steht mit dem erfindungsgemäß eingesetzten Schwammkollagen als Bestandteil von Filmen überraschend ein technologisch sehr einfach zu verarbeitender Rohstoff zur Verfügung, der für das gezielte Colon-Targeting und die Freisetzung im Colon geeignet ist. Im Hinblick auf die veränderte Wirkstoffpassgage könnte angenommen werden, dass der erfindungsgemäße in vivo- eingesetzte Filmbildner die Permeabilität der Darmschleimhaut verringert und dadurch den Organismus zusätzlich vor pathogenen Keimen, Metaboliten oder Toxinen und selbst vor Darm-assoziierten Toxinen und Allergenen, auch aus der Nahrung, schützen kann. Da letztere z.B. als Ursache von Neurodermitis und anderen Erkrankungen von Haut- und Schleimhäuten, auch Nahrungsmittelunverträglichkeiten, asthmatischen Beschwerden, COPD u.a. diskutiert werden, ist auch hier eine Anwendung möglich.

### Abbildungen 1-8

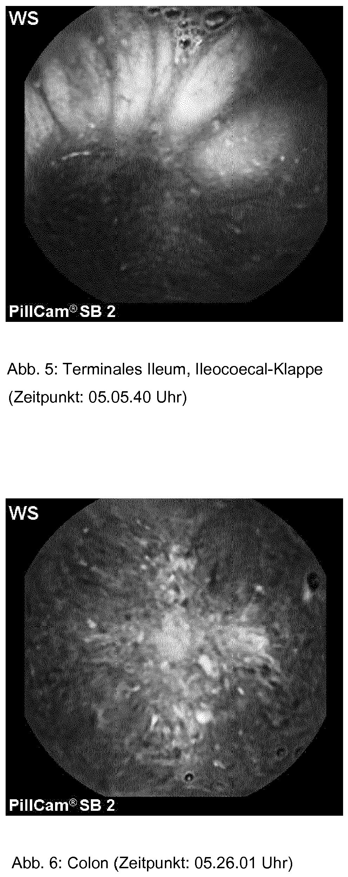

Abb. 1 -8 zeigen die Wirkstoffpassage einer erfindungsgemäß umhüllten Zubereitung gemäß Beispiel **4** nach in vivo-Einnahme über den Magen (Abb.1), Dünndarm (Abb. 2,3; 2 Zeitpunkte), terminales Ileum (Abb. 4,5; 2 Zeitpunkte), Colon (Abb. 6-8; 3 Zeitpunkte). Deutlich ist zu sehen, dass zunächst eine Kollagenfaserverdichtung unter Gelbildung erfolgt, die erst am Ende der Passage ab dem Bereich des terminalen Ileums und zu Beginn des Colons aufbricht und den Wirkstoff rasch und relativ vollständig freigibt, so dass anders als bei der kontinuierlichen Abgabe zu einem bestimmten Zeitpunkt eine spontan-hohe Wirkstoffkonzentration lokal und im Blutkreislauf vorhanden ist (analog der sog Stoßtherapie).

### Nähere Beschreibung der Erfindung

Die vorliegende Erfindung betrifft insofern eine in vivo- Zubereitung zur kontrollierten Colon-Freisetzung zur Prävention und/oder Behandlung entzündlicher und/oder ZNS-abhängiger Erkrankungen, umfassend eine Matrix aus Trägerstoff/en, Zusatzstoffen hierfür und bevorzugt einen oder mehrere arzneilich wirksame Bestandteile (Wirkstoff) sowie einen magensaftresistenten insbesondere externen oder integrierten Überzug, wobei erfindungsgemäß dieser Überzug Schwammkollagen ist und insbesondere in einer Menge von 0,2 bis 10 Gew.%, bezogen auf das Gesamtgewicht der Zubereitung, auf oder in der Matrix vorhanden ist. Der Überzug ist aufgetragen bzw. eingearbeitet/eingemischt aus einer Wasser oder Ethanol-Wasser-haltigen Lösung oder Suspension eines marinen Schwammkollagens, die einen pH- Wert von ca. 6,5 bis 8,5 aufweist. Vorzugsweise ist das marine Schwammkollagen ausgewählt aus Meeresschwämmen der Klasse der Demospongiae und deren 11 Ordnungen Geodiidae, Chondrosiidae, Suberitidae, Axinellidae, Halichondriidae, Dysideidae, Songiidae oder Mischungen hiervon.

Insbesondere enthält die das Schwammkollagen enthaltende Überzugslösung/- Suspension keine weiteren, vor allem magensaftresistenten Retardierungsmittel, wie z. B. ausgewählt aus (Poly)Acrylaten, Cellulosederivaten, Polyvinylalkoholen oder Derivaten hiervon.

Die Matrix liegt vor allem in Form einer Tablette, einer Kapsel, eines Granulates oder eines Dragees vor und der Trägerstoff oder ein Teil hiervon ist vor allem Schwammkollagen, insbesondere marinen Ursprungs.

Als Wirkstoffe eignen sich insbesondere Lecithin aus Ei, oder Soja, Antioxidantien, wie Coenzym Q10, reduziertes Glutathion, Hormone, insbesondere Progesteron, Grünteeextrakte u.a. und/oder vor allem kurzkettige physiologische Säuren und/oder deren Salze/oder Ester, wie Fumarsäure oder Natriumbutyrat.

Derartige Zubereitungen sind vor allem geeignet zur Prävention oder Behandlung von Colitis Ulcerosa, Neurodermitis, Psoriasis, Hepatitiden, mitochondrialen Erkrankungen wie z.B. Rhabdomyolyse, Optikusneuropathie und zahlreichen anderen Schädigungen von Nervenzellen, Nervenfasern, ihren Myelinschichten u.a., oder bei metastasierendem Coloncarcinom bzw. zur Behandlung von Multipler Sklerose und anderen neurodegenerativen Erkrankungen.

Die Erfindung betrifft demnach auch ein Verfahren zum Colon-Targeting von Wirkstoffen, das dadurch gekennzeichnet ist, dass man eine Zubereitung mit kontrollierter Wirkstofffreisetzung im Colon herstellt, indem man eine Matrix, umfassend einen oder mehrere Trägerstoffe wie oben beschrieben, einen oder mehrere Zusatzstoffe hierfür sowie insbesondere einen oder mehrere arzneilich/pharmakologisch wirksame Bestandteile, auf an sich bekannte Weise bereitet und diese Matrix mit einer Wasser oder Ethanol-Wasser-haltigen Lösung oder Suspension eines marinen Schwammkollagens, die einen pH- Wert von ca. 6,5 bis 8,5 aufweist, in ei-ner Menge von 0,2 bis 10 Gew. %, bezogen auf das Gesamtgewicht der Zubereitung, umhüllt, oder alternativ mischt und so eine magensaftresistente Zubereitung mit einem definierten Freisetzungsprofil im Colon erhält.

Die Überzugsuspension ist bevorzugt zusammengesetzt aus 0,1 Gew.% bis 10 Gew.%, vorzugsweise 1 bis 10 Gew.% Schwammkollagen, und als Rest Wasser oder Wasser - Alkohol (vor allem) Ethanol-Gemische mit Zusatzstoffen wie nachfolgend genannt, insbesondere pH-Wertregulatoren, Viskositätsregulatoren, und/oder Konservierungsmittel(n), z. B. 20 oder 25 % bis 70 oder bis 75% (Teile, Gew.%) Wasser, 75 oder 70% bis 25 oder bis 30 Gew.%, vor allem bis 28 Gew.% Ethanol, weiterhin geeignete Mengen an einem oder mehreren pH-Wert-Regulatoren wie Di-Ammoniumhydrogenphosphate, Ammoniumcarbonat, Ammoniak oder Phosphorsäure zur wirkstoffabhängigen Einstellung des optimalen pH-Werts, und ggf. einem oder mehreren Viskositätsregulatoren, und/oder Konservierungsmittel(n).

Die Erfindung umfasst daher auch die Verwendung einer wässrigen oder wässrig-alkoholischen Lösung von marinem Schwammkollagen mit einem pH-Wert von 6,5 bis 8,5 (bei RT), enthaltend ein Puffersystem, ausgewählt aus Phosphatpuffer, Ammoniumhydroxid, Ammoniumcarbonat-Puffern oder weiteren bevorzugt flüchtigen Basen, als magensaftresistentes Überzugsmittel für Zubereitungen mit vor allem arzneilich oder pharmakologisch wirksamen Bestandteilen zum Colon-Targeting für die kontrollierte in vivo-Freisetzung des/der genannten wirksamen Bestandteile.

### Beschreibung der einzelnen Komponenten der erfindungsgemäßen Zubereitung

### 1) Schwammkollagen

Dieses wird gewonnen aus Schwämmen, vorzugsweise Meeresschwämmen, vor allem solchen aus der Klasse der Demospongiae und deren 11 Ordnungen, z.B. den Geodiidae, Chondrosiidae, Suberitidae, Axinellidae, Halichondriidae, Dysideidae, Songiidae und anderen bekannten Vertreten. Es liegt insbesondere nativ (nicht wärme- oder enzymatisch behandelt) vor.

Verfahren zur Gewinnung von Kollagen, welches als Überzugsmittel gemäß vorliegender Erfindung geeignet ist, sind bekannt und beispielsweise einleitend oben genannt, siehe Diehl/Seufert, oder EP 1259 120 B1. Besonders geeignet ist Schwammkollagen, das dadurch hergestellt ist, dass man das Ausgangsmaterial in Alkohol einlegt, anschließend wäscht, mit einem Extraktionsmittel behandelt und den so erhaltenen Kollagenextrakt aufarbeitet. Die Aufarbeitung kann z.B. eine Zentrifugation und das Ausfällen im leicht sauren Bereich mit geeigneten Puffer-und Säuresystemen sein. Einzelheiten sind in der EP 1259120 B1 beschrieben und bekannt. Als Alkohol eignet sich insbesondere Ethanol, als Extraktionsmittel basische Puffersysteme, wie Tris- Puffer. Die erhaltene Kollagen-Suspension kann als solche sofort verwendet werden. Sie kann alternativ gefriergetrocknet/ granuliert und bei Bedarf in Wasser gelöst werden.

Je nachdem, wird der Lösung ein Puffer/Säure-Base-Gemisch, also pH-Wert-Regulatoren, ggf, auch Viskositätsregler wie Wasser, Pektin, u.U. Lecithin und/oder Konservierungsmittel wie niedrig konzentrierte Wasserstoffperoxidlösung zugesetzt, derart, dass der pH-Wert der Kollagenlösung 6 bis 9,5 insbesondere 6,5 bis 8,5, vor allem 6,8 bis 7,5 beträgt. Als pH- Regulator- Puffersysteme eignen sich vor allem Di-/Ammoniumhydrogenphosphate, NH₄-OH (3 bis 30%) oder Ammoniumcarbonat, Ammoniaklösung und Phosphorsäure oder andere vergleichbare Puffersysteme.

Bevorzugt ist das Schwammkollagen der Überzugszubereitung nativ, wie z. B. in der EP 1 259 120 B1 beschrieben.

Die anmeldungsgemäße Überzugszubereitung ist demnach vorzugsweise zusammengesetzt (Mengenangaben/Gew.% bezogen auf die Menge/Gewicht der Gesamtzusammensetzung des Überzugsmittels) aus 0,2 bis 10 Gew.% marinem Schwammkollagen, insbesondere nativem marinem Schwammkollagen aus Schwämmen der eingangs genannten Spezies, bzw. derartigen Fasern, vor allem solchem der Klassen Geodiidae, Chondrosiidae, Axinellidae, insbesondere Chondrosiidae, Wasser oder Wasser-Alkohol-Gemischen, insbesondere Wasser-Ethanol-Gemischen wie 1:8 bis 8:1, z.B. in Mengen von bis 98 Gew.%, pH-Wert-Regulatoren, und ggf. Viskositätsregulatoren und/oder Konservierungs-mitteln in geeigneten Mengen. Weitere Bestandteile (z. B. Talkum) sind hier nicht erforderlich, so dass die anmeldungsgemäße Überzugszubereitung vorzugsweise frei von vor allem Talkum ist. Die so gestaltete wässrige Schwammkollagen-Lösung (oder) /Suspension (oder) / Dispersion oder Kombinationen hiervon wie Lösung mit suspendierten Substanzen mit voreingestelltem pH- Wert wird durch Sprühen z. B. mit einem handelsüblichen Farbsprühgerät vor allem bei Raumtemperatur auf die wirkstoffhaltige Zubereitungsmatrix aufgetragen. Dabei reicht eine Menge aus, welche 0,2 bis 10%, vor allem 0,5 bis 5 Gew.%, bezogen auf die Gesamtmenge der Zubereitung als Umhüllung ergibt

Ggf. kann es von Vorteil sein, der Schwammkollagenlösung 0,1 bis 10% Lecithin z, B. Soja- oder Eilecithin beizufügen.

Andere Überzugsmittel wie die eingangs genannten (magensaftresistenten) Polyacrylate, Hydroxypropylmethylcellulose, Polyvinylalkohole, Vinylacetate oder ähnliche Substanzen sind nicht erforderlich und werden erfindungsgemäß vorzugsweise nicht eingesetzt. Die erfindungsgemäße Zubereitung weist daher als Überzugsmittel Schwammkollagen, ggf. zusammen mit einem oder mehreren Lecithinen auf. Andere Komponenten im Überzug wie hierfür übliche pharmazeutisch verträgliche Farbstoffe und/oder Geschmacksstoffe sind nicht erforderlich und daher bevorzugt nicht vorhanden.

Die Menge an Überzugsmittel ist so gewählt, dass weniger als 30 Gew.% Schwammkollagen, bezogen auf die Gesamtzubereitung, aufgetragen oder vorhanden sind. Erfindungsgemäße Mengen sind von 0,2 bis 10 Gew.%, insbesondere 0,5 bis 5 Gew.%, und ganz besonders 0,5 bis 2,5, vor alle 2 Gew.%.

Die Partikelgröße von Schwammkollagen kann bevorzugt 150 bis 400 nm betragen. Alternativ zum Sprühauftrag kann die Schwammkollagenlösung/-Suspension wie oben beschrieben in analogen Mengen direkt in die Wirkstoffmatrix eingearbeitet sein z. B. durch Vermischen. Bei der Anwendung in vivo (also im wässrigen Schleimhaut-Darmmillieu) entstehen dann auch hier die entsprechenden wie vorstehend genannten Strukturen der Kollagenfasern, welche die beschriebene verzögerte Freisetzung bedingen.

Sofern die Matrix aus Schwammkollagen besteht, ist dieses strukturell vorzugsweise zumindest zum Teil oder vollständig verschieden vom Überzugskollagen wie oben beschrieben. Es kann auch von Vorteil sein, die Matrix vollständig aus Kollagenfasern zu bereiten, in die dann weitere Wirkstoffe/Zusatzstoffe eingearbeitet sein können. Für den Fall, dass das Überzugskollagen integriert ist, ist das Matrixkollagen vom Überzugskollagen strukturell verschieden. Insbesondere kann das externe oder integrierte Überzugskollagen nativ und das Matrixkollagen denaturiert oder nanopartikulär sein.

In einer anderen Ausführungsform kann das Überzugskollagen nativ und die Matrix eine Mischung aus Kollagen (z.B. ausgewählt aus denaturiertem, nativem, nanopartikulärem Kollagen oder Mischungen hiervon), jeweils vor allem aus Schwämmen wie oben beschrieben, oder Kollagen wie genannt (denaturiert, nativ, nanopartikulär oder Mischungen hiervon) in Kombination mit anderen nachfolgend genannten Matrixmaterialien (z. B. Magnesiumcarbonat) sein.

### 2) Wirkstoffmatrix

Als Matrix eignen sich Träger wie nachfolgend beschrieben und Zusatzstoffe hierfür, welche für pharmakologisch /pharmazeutische Produkte, nämlich Medizinprodukte und Arzneimittel, Nahrungsergänzungsmittel, bekannt sind. Basis für diese Matrizes können z. B. sein: Trägerstoffe, ausgewählt aus Kollagen/Kollagenfasern/ (und/oder) Kollagenparti-keln, insbesondere Schwammkollagen wie oben beschrieben, nämlich vor allem marines Schwammkollagen aus der Klasse der Demospongiae und deren 11 Ordnungen, z.B. den Geodiidae, Chondrosiidae, Suberitidae, Axinellidae, Halichondriidae, Dysideidae, Songiidae; weiterhin daraus durch Erwärmung oder enzymatische Behandlung entstandenes denaturiertes Schwammkollagen aus Meeresschwämmen, der Art wie oben genannt, oder daraus erhaltenes nanopartikuläres Schwammkollagen wie aus der EP 1259120 B1 bekannt (vernetztes Kollagen, z. B. mit Glutaraldehyd vernetzt).

Denaturiertes Kollagen oder Fasern, Partikel hiervon sind säureunlöslich und können zur Weiterverarbeitung in basischer wässriger Lösung (Basen wie Alkylamine wie (Mono, Di/-Tert.)Methyl/Ethyl/Propylamin, Salze wie Phosphat- oder Ammoniumsalze ggf. unter Hinzufügen von Lösungsmitteln/Trennmitteln wie Toluol, Alkohole wie Isopropanol, Ethanol gelöst werden.

Kollagen oder vor allem auch denaturiertes Schwammkollagen wie oben beschrieben ist dann sowohl Träger oder Teil der Trägerstoffe, welcher gleichzeitig auch pharmakologische, insbesondere entzündungshemmende, und/oder anti-oxidative Wirkungen entfalten kann (also insofern auch Wirkstoff). Derartiges marines Schwammkollagen (nativ oder denaturiert) ist als Träger oder Teil der Trägermasse (z.B. bis 15 Gew%) besonders bevorzugt, vor allem in Kombination mit Fumarsäure, einem Esterderivat hiervon wie Dimethylester, Antioxidantien, Coenzym Q 10, und/oder einem oder mehreren Lecithinen als Wirkstoff(e).

Weitere geeignete Trägerstoffe sind: Calciumchlorid, Magnesiumchlorid, Natriumbicarbonat, oder auch Kombinationen hiervon mit dem zuvor genannten Schwammkollagenträger z. B. in Mengen von 0,5 bis 15 Gew.%.

Übliche Matrix-Zusatzstoffe sind ausgewählt aus Bindemitteln wie Pektin, Talkum, Magnesiumstearat, Salzen, Basen, bekannten Sprengmitteln, Fließregulierungsmitteln und Schmiermitteln. Dazu gehören z. B. mikrokristalline Cellulose, hochdisperses Siliciumdioxid, Pulvercellulose, Calciumhydrogenphosphat-Derivate, Laktose, Mannitol, Stärke z. B. aus Reis, Mais, Kartoffeln, und analoge, dem Fachmann für diese Zwecke bekannte Mittel.

### 3)Wirkstoffe

Hierzu gehören enteral, insbesondere oral verabreichbare Wirkstoffe für unterschiedlichste Indikationen, insbesondere solche, die zur Behandlung von ZNS-abhängigen, entzündlichen Erkrankungen eingesetzt werden ebenso wie Erkrankungen, insbesondere entzündliche Erkrankungen des Darms, des Muskelsystems, des Skeletts, der Haut, der Nervenzellen und deren Fasern, neurodegenerativen Erkrankungen, Morbus Alzheimer, Querschnittslähmung, Colitis Ulcerosa (CU) und Colon Carcinom, Morbus Crohn, Hepatitiden, Multiple Sklerose, Neurodermitis, Nahrungsmittelallergien, Psoriasis. Eine nicht abschließende Auswahl an Wirkstoffen umfasst: Lecithin z.B. von Soja, Ei, Phosphatidylcholin zur Behandlung Colitis Ulcerosa; Entzündungen des Bewegungsapparates (z. B. mit denaturiertem Schwammkollagen als Träger/ Wirkstoff). Insbesondere geeignet für erfindungsgemäße Zubereitungen sind Wirkstoffe mit einer Säure- und/oder Carboxylfunktion wie Fumarsäure (z. B. zur Behandlung von Multipler Sklerose), Phosphatidylcholin (z.B. bei Colitis Ulcerosa), Natriumbutyrat, Orotsäure, Phenylessigsäurederivate (z. B. nicht steroidale Antiphlogistika) wie Diclophenac, oder auch Ibuprofen, Acetylsalicylsäure, Glutathion, andere Aminosäuren, entzündungshemmende Mittel aus der Gruppe der Hormone, der Bioflavonide wie Rutin, Quercetin, Coenzym Q10, Bioflavonoide aus schwarzen Johannisbeer- oder Citrusfrüchten, Grünteeextrakte und andere natürliche Antioxidantien, Hormone mit ZNS-Schutzeigenschaften wie Progesteron, marines Schwammkollagen, natives marines Schwammkollagen, denaturiertes marines Schwammkollagen.

### Herstellung

Erfindungsgemäße Zubereitungen werden hergestellt, indem man zunächst das Überzugsmittel bereitet. Dazu können gemäß z.B. der EP 1 259 120 B1 oder anderen Verfahren isolierte Kollagenfasern (und/oder)/-partikel, die in einer Menge von 0,1 bis 30, vor allem 0,2 bis 10 Gew.% zusammen mit Wasser oder einem Wasser/Alkohol-Gemisch, im Verhältnis von z.B. 4:1 bis 1:4, wie in einer Menge von 98 bis 70% Gew.%, bezogen auf die Gesamtmenge des Überzugsmittels, z.B. und 90 bis 98 Gew. % eines Gemisches aus Wasser und Ethanol mit 25 bis 70 % Wasser und 75 bis 30% Ethanol, mit pH-Wert-Regulatoren wie flüchtigen basischen Reagenzien, wie z.B. NH₄OH (3 bis 30%) oder Ammoniumcarbonat, basischen Phosphaten (z.B. Di-(und/oder)/Ammoniumhydrogenphosphate, z. B: Diammoniumhydrogenphosphat und/oder Natriumdihydrogenphosphat), u.a. unter Rühren gelöst und auf einen neutralen pH-Wert von z. B. ca. 6,8 bis 7,2 eingestellt werden. Falls erforderlich können noch Mittel zur Reduktion/Einstellung der Viskosität wie Pektin, Wasser, oder Mittel zur Konservierung/Reduktion möglicher Keime wie niedrig konzentrierte Wasserstoffperoxidlösung zugesetzt werden.

Die erhaltene Mischung kann mit geeigneten Sprühgeräten z. B unter Verwendung eines Dragierkessels auf die an sich bekannte Weise bereitete Matrices wie Pellets, Tabletten, Kapseln, Granulate, und auf andere feste Arzneiformen mittels Wirbelschichtgeräten oder einer anderen geeigneten Technologie aufgebracht werden. Derartige Verfahren sind bekannt. Die Pellets, Kapseln, Tabletten, Granulat u.a. können dabei die Matrix enthalten oder selbst die Matrix darstellen.

Bei einem Substanzauftrag von nur 13 mg/cm² (dies sind bis etwa 8 Gew.%, bezogen auf die Gesamtmenge der Zubereitung, insbesondere bei runden bikonvexen Tabletten, Pellets oder Kapseln, werden bereits magensaftresistente Zubereitungsformen erhalten. Damit entsprechen die überzogenen Arzneiformen den Prüfungsvorschriften für magensaftresistente Tabletten nach Ph. Eur..

Zur Herstellung von Zubereitungen mit integriertem Überzug kann das oben genannte Überzugsmittel oder Suspension in die Matrix in der angegebenen Menge eingemischt und die Zubereitung anschließend auf bekannte Weise getrocknet werden.

Sofern die Matrix neben Wirk-und/oder Zusatzstoffen marines Schwammkollagen selbst enthält oder aus diesem neben Wirk-und/oder Zusatzstoffen besteht, kann das Matrix-Kollagen auf die gleiche Art gewonnen werden wie das Überzugskollagen oder es wird vorzugsweise aus denaturiertem Schwammkollagen bereitet. Dazu kann z.B. eine wässrigethanolische Kollagenlösung erhitzt werden. Sie kann auch z.B. gefriergetrocknet und als Matrix so oder in Kombination mit anderen Matrixmaterialien auf an sich bekannte Weise verarbeitet werden zu Granulat, Pellets, Pulver, Tabletten oder anderen Formulierungen.

Insgesamt ist es bevorzugt, wenn das Überzugsmittel, das vor allem natives Schwammkollagen ist, extern auf der Matrix appliziert ist.

Wie erläutert, umfasst die Matrix marines Schwammkollagen, insbesondere denaturiertes Schwammkollagen, zu einem Teil oder besteht daraus. (ggf. neben Wirk-und Zusatzstoffen).

Weiterhin ist es bevorzugt, wenn das Überzugsmittel extern auf der Matrix appliziert ist. Vor allem bevorzugt ist es, wenn die Matrix marines Schwammkollagen, insbesondere denaturiertes und/oder nanopartikuläres Schwammkollagen, zu einem Teil umfasst oder daraus besteht (ggf. neben Wirk-und Zusatzstoffen) und das Überzugsmittel, insbesondere natives Schwammkollagen, extern auf der Matrix appliziert ist.

### Anwendung

Erfindungsgemäß herstellte Zubereitungen werden vor allem zur Behandlung/Prävention von entzündlichen und /oder ZNS-abhängigen Erkrankungen eingesetzt, wobei insbesondere eine höhere lokale Wirkstoffmenge erforderlich sein kann, im Unterschied zu retardierten kontinuierlich freigesetzten Abgabeprofilen. Damit wird eine effektive, der Stoßtherapie vergleichbare Wirkung erzielt. Dies kann z. B. bei Colitis Ulcerosa, Multipler Sklerose, mitochondrialen Erkrankungen, wie auch Rhabdomyolyse, Optikusneuropathie und zahlreichen anderen Schädigungen von Nervenzellen, Nervenfasern, deren Myelinschichten, Hepatitiden, Neurodermitis, Psoriasis oder Coloncarcinomen von Vorteil sein.

Ferner ist es möglich, im Falle von Sepsis oder HIV eine physiologische Barriere aufzubauen, die den Übertritt von Keimen und Toxinen in Gewebe und körpereigene Systeme verhindert. Dazu kann eine Zubereitung mit Schwammkollagen als Matrix mit Wirkstoffeigenschaft (z.B. denaturiert, oder denaturiert und nativ im Gemisch) als Granulat oder verkapselt angewendet werden und ggf. ein stärkeres erfindungsgemäßes Kollagencoating von ca. 10 Gew.% aufgetragen werden.

Andere Indikationen/Wirkstoffe sind: Morbus Alzheimer, Neuropathien (z.B. 50 mg Fumarsäure, zusätzlich z.B. 100 mg Orotsäure oder Coenzym Q10, z. B. in einer Menge 100mg); Orotsäure mit Arginin, oder Ornithin-Aspartat für die Gedächtnisleistung; Querschnittslähmung (jeweils z.B. 50 mg freie Fumarsäure, oder Fumarsäure auch in Kombination mit Coenzym Q10, in Mengen wie vorstehend angegeben); oder Colitis Ulcerosa (CU) bzw. Colon Carcinome (jeweils z.B. 50 mg Natriumbutyrat).

Die Art und Weise der Verabreichung richtet sich nach den jeweiligen Vorgabe, dem Alter, Geschlecht, Gewicht des behandelten Subjekts, insbesondere Menschen. Die Zubereitungen können als Arzneimittel oder als Medizinprodukt oder Ergänzungsmittel je nach pharmazeutischer Vorgabe vorliegen.

Es hat sich gezeigt, dass die Wirkstoffmenge und Art dabei auch im Vergleich zur herkömmlichen Dosierungsformen reduziert bzw. vereinfacht werden kann. Beispielweise kann erfindungsgemäß anstatt Fumarsäuredimethylester die physiologische freie Fumarsäure eingesetzt und diese auf ca. 1/7 der bisher erforderlichen Menge reduziert werden. Bei anderen Wirkstoffen können z.B. Reduktionen von 10 bis 50 % möglich sein. Dies führt zu geringeren Nebenwirkungen und auch zu verkürzten Behandlungszeiträumen.

### Beispiele

### Beispiel 1: Herstellung einer Schwammkollagen-Lösung (3%) als Überzugsmittel

Gemäß z.B. der EP 1 259 120 B1 isolierte native Kollagenfasern aus Schwämmen der Klasse Chondrosia (3 Gew.%) in Wasser-/Ethanol (ca. 37%/60%) wurden mit Diammoniumhydrogenphosphat/Natriumdihydrogenphosphat unter Rühren gelöst und auf einen neutralen pH-Wert von ca. 7,2 eingestellt. Es wurde noch eine geringe Menge Wasser zur Reduktion der Viskosität und zur Konservierung niedrig konzentrierte Wasserstoffperoxidlösung) hinzugesetzt.

### Beispiel 2: Herstellung einer Schwammkollagen-Lösung (1,5%) als Überzugsmittel

Es wurde eine gemäß der o.g. EP 1259120 B1 gewonnene Kollagenlösung aus dem Schwamm der Klasse Dysidia mit Wasser/Ethanol mit Schwammkollagenfasern (nativ) als Ausgangsmaterial zur Herstellung der umhüllenden Filmsuspension(Lösung) hergestellt, welche 38% Ethanol, 60,5 % Wasser, 1,5 % Kollagen sowie Ammoniumhydroxid aufwies. Dieser Lösung wurde Phosphorsäure, Ammoniak in geeigneten Mengen sowie ferner eine geringe Menge Pektin zur Einstellung der Viskosität hinzugesetzt. Der der pH-Wert der Lösung war auf ca. 6,9 eingestellt.

### Beispiel 3: Herstellung einer mit Schwammkollagen-umhüllten Wirkstoffmatrix in Form eines Granulats mit Lecithin als Wirkstoff

Eine Suspension mit 10% Kollagenfasern aus dem Schwamm Axinella (oder alternativ Chondrosia) in Wasser/Ethanol, jeweils ca. 50%), erhalten nach der in der EP 1 259 120 B1 beschriebenen Methode, wurde auf eine Temperatur von ca. 65° C für einen Zeitraum von ca. 15 Minuten erwärmt. Es bildete sich ein denaturiertes säureunlösliches Schwammkollagen. Diese Zubereitung wurde in Gegenwart von flüchtigen Basen oder flüchtigen Salzen, nämlich Di-Natriumhydrogenphosphat gelöst und intensiv gerührt. Der dabei entstandene Schaum wurde unter weiterem Rühren mit 2%, bezogen auf die Gesamtmenge der Suspension, Ei-Lecithin versetzt. Es entsteht ein überwiegend zäher Teig, dem, falls die Konsistenz es erfordert, Verdickungsmittel wie Pektin o.a., Trennmittel, organische wasserschleppende Zusätze, wie Alkohole, Toluole u.a. in geeigneten Mengen beigefügt werden können. Der erhaltene zähe Teig lässt sich dann mit geeigneten Geräten, z.B. von Erweka (D-63130 Heusenstamm), Extrudern, Sieben o.a. granulieren und bei Temperaturen < 50°C trocknen. Sowohl der frisch hergestellte Ansatz (Paste) als auch das getrocknete Gut lassen sich auch in Kapseln abfüllen. Kapseln und Granulat werden anschließend mit definierten Mengen an Kollagenschaum, hergestellt nach Beispiel 1, in einem Dragierkessel oder einem geeigneten Sprühtrockner magensaftresistent überzogen. Die Überzugspartikel in Granulatform als auch die Kapseln waren gemäß den Prüfungsvorschriften nach Ph.Eur. magensaftresistent.

### Beispiel 4: In-vivo Monitoring der Auflösung von Dünndarm-/ Colon-Iöslichen Granulaten oder Kapseln mit Schwammkollagen als Micro- / Nanopartikel, Colon-Targeting

Gemäß der in Beispiel 3 beschriebenen Methode wurden mit Schwammkollagenen überzogene Granulate mit 2(w/w) Kollagenüberzug zusammen mit einer Dünndarm-Kapsel, PillCam SB2, GIVEN IMAGING LTD. Yokneam, Israel, von einem freiwilligen Probanden geschluckt. Es zeigte sich überraschend, dass sich die Granulate erst im Bereich des terminalen Ileum unmittelbar vor dem Eintritt in das Colon in Kollagenfasern aufspalten, und die Arzneimittel (Ei-Lecithin), freigeben. Die Kollagenfasern lagern sich wie ein Film auf die Oberfläche der Dickdarmschleimhaut und geben dort ihre transportierten Wirkstoffe ab. Gegen Ende des Colonbereiches bis zum Rektum beobachtet man zunehmend kürzere Schwammkollagenfasern aufgrund von biologischen Verdauungsprozessen. Demnach löst sich die erfindungsgemäße Zubereitung erst spät im Gastrointestinaltrakt im terminalen Ileum zu Beginn des Dickdarms auf einmal, und nicht kontinuierlich auf. Es handelt sich dabei um ein selektives Colon-Targeting. Dies ist deutlich anhand der Abb.1 (Magen) 2-3 (oberer Dünndarm), 4-6 (Dickdarm) bzw. 7-8 (Colon, terminales Colon) erkennbar.

Die Freisetzung von Substanzen im Colon auf der Basis von Schwammkollagen als insbesondere alleiniger magensaftresistenter Überzug bietet somit eine Fülle von einzigartigen Anwendungsmöglichkeiten für Aktivstoffe und Wirkprinzipien dar. Im Falle von Sepsis oder HIV, oder allergischen Erkrankungen u.a. lässt sich so auch eine physiologische Barriere aufbauen, die den Übertritt von Keimen und Toxinen in Gewebe und körpereigene Systeme verhindert.

### Beispiel 5: Anwendung erfindungsgemäßer Zubereitungen bei Colitis ulcerosa (CU)

Analog Beispiel 3 hergestellte Lecithin-Granulate (mit Schwammkollagen-Matrixgranulat aus der Klasse Geodia bzw. Kapseln mit diesem Granulat mit jeweils einem Überzug aus 2% Schwammkollagen, wurden 39 Patienten mit CU verordnet und der Erkrankungsverlauf mit Hilfe eines strukturierten Fragebogens über 6 Wochen dokumentiert. Sowohl Kapseln als auch Granulat führten zu einer Besserung der klinischen Symptome.

Am Ende der Therapiephase waren 22% beschwerdefrei, 36% hatten sich deutlich gebessert, 32% hatten sich verbessert und 10% waren unverändert. Die Wirkung trat im Mittel nach der dritten Woche ein.

### Beispiel 6: Anwendung bei Multipler Sklerose (MS)

Nach Beispiel 3 hergestellte und mit Schwammkollagenen überzogene Granulate bzw. Kapseln mit Schwammkollagen (Matrixkollagen aus Chondrosia), Lecithin (400 mg) und als weiteren Bestandteil entweder Fumarsäuredimethylester bzw. freie Fumarsäure (30 mg, bzw. 50 mg) wurden Patienten mit MS verordnet und der Erkrankungsverlauf mit Hilfe eines strukturierten Fragebogens über 8 Wochen dokumentiert. Sowohl Kapseln als auch Granulat mit Fumarsäure als Ester und - besonders überraschend - die physiologische freie Fumarsäure allein führten zu einer Besserung der klinischen Symptome, besonders der motorischen Beschwerden. So waren Patienten bereits nach wenigen Dosierungen (über das Wochenende) wieder in der Lage, selbst mit Messer und Gabel zu essen. Eine völlig pflegebedürftige MS Patientin konnte nach 14 tägiger dreimal täglicher Einnahme der erfindungsgemäßen Formulierungen mit zunächst 30mg, dann 50mg freier Fumarsäure wieder selbständig mit Messer und Gabel essen, im Gehwagen gehen, Fahrradübungen durchführen und alleine zu Bett gehen.

Die erfindungsgemäße besondere Galenik ermöglichte eine Therapie mit deutlich niedrigeren Wirkstoff-Konzentrationen als ein in der Entwicklung befindliches Fumarsäure-Ester-Präparat, Tecfidera®, ein Fumaderm®-Analogon. Um klinisch wirksame Effekte zu erzielen sind bei diesem Retard-Präparat mit herkömmlicher Galenik Konzentrationen von 250 mg pro Kapsel erforderlich. Mit der erfindungsgemäßen Colon-Targeting- und Colon-Freisetzungs- Technologie reichen dagegen nur 30 bis 50 mg pro einzelner Dosis aus, um innerhalb weniger Tage bereits einen deutlichen klinischen Effekt zu erzielen.

### Beispiel 7: Anwendung bei Neurodermitis

Nach Beispiel 3 hergestellte und mit 2% Schwammkollagenen überzogene Granulate (Matrix: Axinella) mit 400 mg Ei-Lecithin wurden 3 Patienten mit Neurodermitis verabreicht und der Erkrankungsverlauf mit Hilfe eines strukturierten Fragebogens über 6 Wochen dokumentiert. Innerhalb eines Monats kam es zu einer deutlichen Besserung der klinischen Symptome.

### Beispiel 8: Weitere Anwendungen bei Neurodegenerativen Erkrankungen, Morbus Alzheimer, Querschnittslähmung, Hepatitis C, CU und Colon Carcinom mit zusätzlich 50mg freier Fumarsäure oder 50mg Natriumbutyrat

Nach Beispiel 3 hergestellte und mit 1 %Schwammkollagenen überzogene Granulate oder Kapseln mit Schwammkollagen aus Axinella (bzw. Chondrosia), Lecithin und als weiteren Bestandteil entweder 50mg freie Fumarsäure oder 50mg Natriumbutyrat wurden Patienten mit neurodegenerativen bzw. entzündlichen Erkrankungen, nämlich Morbus Alzheimer (50mg Fumarsäure, zusätzlich 100mg Orotsäure, oder Orotsäure mit Arginin, oder Ornithin-Aspartat, Coenzym Q10), bzw. Querschnittslähmung (jeweils 50mg freie Fumarsäure), bzw. bei Hepatitis C (Zubereitung gemäß Beispiel 3) mit 250mg Reduziertes Glutathion), CU bzw. Colon Carcinomen (jeweils 50mg Natriumbutyrat) verabreicht. Der Erkrankungsverlauf wurde mit Hilfe eines strukturierten Fragebogens über 8 Wochen dokumentiert. Sowohl Kapseln als auch Granulat mit freier Fumarsäure bzw. Natriumbutyrat führten zu einer Besserung der neurologischen klinischen Symptome, der motorischen oder kognitiven Beschwerden und depressiven Stimmungsschwankungen. Auch bei CU und Colon Carcinom-Patienten konnte bei der Coloskopie ein Rückgang entzündlicher Veränderungen der Darmschleimhaut festgestellt werden.

Die erfindungsgemäße besondere Galenik ermöglichte somit eine positive Beeinflussung selbst neurologischer und ZNS lokalisierter Symptome durch Colon Freisetzung (Colon Targeting und Colon Release) durch niedermolekulare physiologische Stoffwechselmetabolite in unerwartet niedrigen Wirkstoff-Konzentrationen.

## Patentansprüche

1. In vivo- Zubereitung zur kontrollierten Colon-Freisetzung aus einer festen bis pastenförmigen Matrix mit einem oder mehreren Trägerstoff/en, ggf. Zusatzstoffen hierfür und einen oder mehrere Wirkstoffe oder eine diese Matrix enthaltende Verabreichungsform, sowie ein magensaftresistentes Überzugsmittel, **dadurch gekennzeichnet, dass** dieses Überzugsmittel ein Talkum-freies marines Schwammkollagen ist, welches entweder extern auf der Matrix oder darin integriert als Teil des davon zumindest teilweise verschiedenen Trägers, in einer Menge von 0,2 bis 10 Gew.%, bezogen auf das Gesamtgewicht der Zubereitung aufgetragen oder eingemischt ist, wobei die Matrix marines denaturiertes Schwammkollagen als Träger oder als Teil der Trägermasse aufweist, welches strukturell verschieden ist vom Überzugskollagen.

2. In vivo- Zubereitung zur kontrollierten Colon-Freisetzung nach Anspruch 1, **dadurch gekennzeichnet, dass** der Überzug, auf der Matrix vorhanden ist, und aufgetragen ist aus einer Wasser oder Ethanol-Wasser-haltigen Lösung oder Suspension eines marinen Schwammkollagens, die einen pH- Wert von ca. 6,5 bis 8,5 aufweist.

3. Zubereitung nach einem der Ansprüche 1 oder 2 , **dadurch gekennzeichnet, dass** das marine Schwammkollagen ausgewählt ist aus Meeresschwämmen der Klasse der Demospongiae und deren 11 Ordnungen Geodiidae, Chondrosiidae, Suberitidae, Axinellidae, Halichondriidae, Dysideidae, Songiidae oder Mischungen hiervon.

4. Zubereitung nach einem der Ansprüche 1 bis 3, **dadurch gekennzeichnet, dass** das das Schwammkollagen enthaltende Überzugsmittel keine weiteren magensaft-resistenten Retardierungsmittel, (z. B. ausgewählt aus (Poly)Acrylaten, Cellulosederivaten, Polyvinylalkoholen oder Derivaten hiervon) enthält.

5. Zubereitung nach einem der Ansprüche 1 bis 4, **dadurch gekennzeichnet, dass** die Matrix oder eine diese enthaltende Verabreichungsform in Form einer Tablette, eines Granulates, einer Kapsel oder eines Dragees vorliegt und mindestens ein Teil des Trägerstoffs denaturiertes marines Schwammkollagen ist.

6. Zubereitung nach einem der Ansprüche 1 bis 5, **dadurch gekennzeichnet, dass** als Überzug natives Schwammkollagen in einer Menge von 0,5 bis 9 Gew.%, bezogen auf das Gesamtgewicht der Zubereitung, auf der Matrix vorhanden ist.

7. Zubereitung nach einem der Ansprüche 1 bis 6 , **dadurch gekennzeichnet, dass** der Wirkstoff ausgewählt ist aus Lecithin aus Ei, Soja, Fumarsäure, Natriumbutyrat, Coenzym Q 10, Glutathion, Orotsäure, Arginin, Progesteron oder Ornithin oder Kombinationen hiervon ist.

8. Zubereitung nach einem der Ansprüche 6 oder 7 , **dadurch gekennzeichnet, dass** der Wirkstoff Fumarsäure, Lecithin, Coenzym Q10 oder Kombinationen hiervon ist.

9. Zubereitung nach einem der Ansprüche 1 bis 8 zur Anwendung bei der Prävention oder Behandlung von Multipler Sklerose oder Psoriasis, wobei als Wirkstoff Fumarsäure enthalten ist.

10. Zubereitung nach einem der Ansprüche 1 bis 8 zur Anwendung bei der Prävention oder Behandlung von Colitis Ulcerosa, wobei der Wirkstoff Lecithin ist.

11. Verfahren zur Herstellung einer Zubereitung mit kontrollierter Wirkstofffreisetzung im Colon (Colon-Targeting), **dadurch gekennzeichnet, dass** man eine Matrix oder eine diese enthaltende Verabreichungsform, umfassend einen oder mehrere Trägerstoffe, umfassend marines Schwammkollagen, einen oder mehrere Zusatzstoffe hierfür sowie einen oder mehrere Wirkstoffe, auf an sich bekannte Weise bereitet und diese Matrix mit einer Wasser oder Ethanol-Wasser-haltigen Lösung oder Suspension eines marinen Schwammkollagens, die einen pH- Wert von ca. 6,5 bis 8,5 aufweist, in einer Menge von 0,2 bis 10 Gew.%, bezogen auf das Gesamtgewicht der Zubereitung, mischt oder umhüllt und so eine magensaftresistente Zubereitung mit einem definierten Freisetzungsprofil im Colon erhält, wobei die Matrix marines denaturiertes Schwammkollagen als Träger oder als Teil der Trägermasse aufweist, welches strukturell verschieden ist vom Überzugskollagen.

12. Verfahren nach Anspruch 11, **dadurch gekennzeichnet, dass** das Schwammkollagen ausgewählt ist aus der Klasse der Demospongiae und die Überzugsuspension zusammengesetzt ist aus 2 bis 10 % Schwammkollagen, und 90 bis 98 Gew. % eines Gemisches aus Wasser und Ethanol mit 25 bis 70 % Wasser und 75 bis 30% Ethanol, umfassend weiterhin eine oder mehrere pH-Wert-regulierenden Substanzen, ausgewählt aus Di-Ammoniumhydrogenphosphat, Di-Ammoniumcarbonat, NH₄OH, Ammoniaklösung und Phosphorsäure, zur optimalen pH-Wert- Einstellung, einen oder mehrere Viskositätsregulierer, und/oder ein oder mehrere Konservierungsmittel.

13. Verfahren nach Anspruch 11 oder 12, **dadurch gekennzeichnet, dass** die Matrix oder eine diese enthaltende Verabreichungsform eine Tablette, ein Granulat oder eine Kapsel und der Wirkstoff Lecithin, Coenzym Q10, und/oder Fumarsäure ist.

14. Verfahren nach einem der Ansprüche 11 bis 13, **dadurch gekennzeichnet, dass** die Schwammkollagen-Überzugslösung/-Suspension auf die Matrix aufgesprüht wird.

15. Verwendung einer Zubereitung gemäß einem der Ansprüche 1 bis 7 zur Herstellung eines Mittels zur Prävention oder Behandlung von Morbus Alzheimer, Neuropathien, **dadurch gekennzeichnet, dass** die Zubereitung als Wirkstoff Fumarsäure, zusätzlich Orotsäure oder Coenzym Q10 aufweist.

16. Verwendung einer Talkum-freien wässrigen oder wässrig-alkoholischen Lösung oder Suspension von marinem Schwammkollagen mit einem pH-Wert von 6,5 bis 8,5, enthaltend pH-Wert-Regulatoren, insbesondere ausgewählt aus Phosphatpuffer, Ammoniumhydroxid, Ammoniumcarbonat-Puffern und/oder Ammoniaklösung und Phosphorsäure, als magensaftresistentes externes oder integriertes Überzugsmittel zur Herstellung von Zubereitungen mit Wirkstoffen zum Colon-Targeting für die kontrollierte in vivo-Freisetzung des/der Wirkstoffe oder für den in vivo- Aufbau einer physiologischen Barriere im Colon zur Verhinderung des Übertritts von Keimen und Toxinen in Gewebe und körpereigene Systeme eines Säugetiers, insbesondere des Menschen, wobei die Matrix der Zubereitung marines denaturiertes Schwammkollagen als Träger oder als Teil der Trägermasse aufweist, welches strukturell verschieden ist vom Überzugskollagen.

## Claims

1. An *in vivo* preparation for controlled colonic release from a solid to pasty matrix comprising one or more carrier substance(s), optionally additives therefor and one or more active ingredients or an administration form containing said matrix together with a gastric juice-resistant coating composition, **characterised in that** said coating composition is a talcum-free, marine sponge collagen which is either applied externally onto the matrix or incorporated therein as part of the carrier which at least partially differs therefrom in a quantity of 0.2 to 10 wt.%, based on the total weight of the preparation, wherein the matrix comprises marine denatured sponge collagen as carrier or as part of the carrier composition, which collagen differs structurally from the coating collagen.

2. An *in vivo* preparation for controlled colonic release according to claim 1, **characterised in that** the coating is present on the matrix and is applied from a solution or suspension, containing water or ethanol and water, of a marine sponge collagen, which solution or suspension has a pH of approx. 6.5 to 8.5.

3. A preparation according to one of claims 1 or 2, **characterised in that** the marine sponge collagen is selected from marine sponges of the class Demospongiae and the 11 orders thereof Geodiidae, Chondrosiidae, Suberitidae, Axinellidae, Halichondriidae, Dysideidae, Spongiidae or mixtures thereof.

4. A preparation according to one of claims 1 to 3, **characterised in that** the coating composition containing the sponge collagen contains no further gastric juice-resistant controlled-release agents (for example selected from (poly)acrylates, cellulose derivatives, polyvinyl alcohols or derivatives thereof).

5. A preparation according to one of claims 1 to 4, **characterised in that** the matrix or an administration form containing it assumes the form of a tablet, a granular product, a capsule or a sugar-coated tablet and at least part of the carrier substance is denatured marine sponge collagen.

6. A preparation according to one of claims 1 to 5, **characterised in that** native sponge collagen is present as a coating on the matrix in a quantity of 0.5 to 9 wt.%, based on the total weight of the preparation.

7. A preparation according to one of claims 1 to 6, **characterised in that** the active ingredient is selected from egg or soy lecithin, fumaric acid, sodium butyrate, coenzyme Q10, glutathione, orotic acid, arginine, progesterone or ornithine or combinations thereof.

8. A preparation according to one of claims 6 or 7, **characterised in that** the active ingredient is fumaric acid, lecithin, coenzyme Q10 or combinations thereof.

9. A preparation according to one of claims 1 to 8 for use in the prevention or treatment of multiple sclerosis or psoriasis, wherein fumaric acid is present as the active ingredient.

10. A preparation according to one of claims 1 to 8 for use in the prevention or treatment of ulcerative colitis, wherein the active ingredient is lecithin.

11. A method for producing a preparation with controlled active ingredient release in the colon (colon targeting), **characterised in that** a matrix or an administration form containing said matrix comprising one or more carrier substances, comprising marine sponge collagen, one or more additives therefor and one or more active ingredients is prepared in a manner known *per se* and said matrix is mixed or enveloped with a solution or suspension containing water or ethanol and water of a marine sponge collagen, which solution or suspension has a pH of approx. 6.5 to 8.5, in a quantity of 0.2 to 10 wt.%, based on the total weight of the preparation, and a gastric juice-resistant preparation with a defined release profile in the colon is obtained in this manner, wherein the matrix comprises marine denatured sponge collagen as carrier or as part of the carrier composition, which collagen differs structurally from the coating collagen.

12. A method according to claim 11, **characterised in that** the sponge collagen is selected from the class of Demospongiae and the coating suspension is composed of 2 to 10% sponge collagen, and 90 to 98 wt.% of a mixture of water and ethanol comprising 25 to 70% water and 75 to 30% ethanol, furthermore comprising one or more pH-regulating substances selected from diammonium hydrogen phosphate, diammonium carbonate, NH₄OH, ammonia solution and phosphoric acid for establishing an optimum pH, one or more viscosity regulators, and/or one or more preservatives.

13. A method according to claim 11 or 12, **characterised in that** the matrix or an administration form containing said matrix is a tablet, a granular product or a capsule and the active ingredient is lecithin, coenzyme Q10, and/or fumaric acid.

14. A method according to one of claims 11 to 13, **characterised in that** the sponge collagen coating solution/suspension is sprayed onto the matrix.

15. Use of a preparation according to one of claims 1 to 7 for producing an agent for the prevention or treatment of Alzheimer's disease or neuropathies, **characterised in that** the preparation comprises fumaric acid, additionally orotic acid or coenzyme Q10 as active ingredient.

16. Use of a talcum-free aqueous or aqueous-alcoholic solution or suspension of marine sponge collagen with a pH of 6.5 to 8.5, containing pH regulators, in particular selected from phosphate buffer, ammonium hydroxide, ammonium carbonate buffer and/or ammonia solution and phosphoric acid, as an external or integrated gastric juice-resistant coating composition for producing preparations comprising active ingredients for colon targeting for controlled *in vivo* release of the active ingredient(s) or for *in vivo* synthesis of a physiological barrier in the colon for preventing transfer of microorganisms and toxins into the tissue and endogenous systems of a mammal, in particular of a human, wherein the matrix of the preparation comprises marine denatured sponge collagen as carrier or as part of the carrier composition, which collagen differs structurally from the coating collagen.

## Revendications

1. Préparation in vivo pour une libération contrôlée dans le côlon à partir d'une matrice solide à pâteuse comprenant un ou plusieurs excipients, éventuellement des additifs à cette fin et un ou plusieurs principes actifs ou une forme posologique contenant cette matrice, ainsi qu'un agent d'enrobage résistant au suc gastrique, **caractérisée en ce que** cet agent d'enrobage est un collagène d'éponge marin exempt de talc, lequel est soit appliqué de manière externe sur la matrice, soit mélangé de manière intégrée dans celle-ci en tant que partie du support au moins en partie différent de celle-ci, dans une quantité de 0,2 à 10 % en poids, rapporté au poids total de la préparation, dans laquelle la matrice comprend du collagène d'éponge marin dénaturé en tant que support ou en tant que partie de la matière de support, lequel est différent d'un point de vue structural du collagène d'enrobage.

2. Préparation in vivo pour une libération contrôlée dans le côlon selon la revendication 1, **caractérisée en ce que** l'enrobage est présent sur la matrice et est appliqué à partir d'une solution ou suspension contenant de l'eau ou de l'éthanol-eau d'un collagène d'éponge marin qui présente une valeur de pH d'environ 6,5 à 8,5.

3. Préparation selon l'une des revendications 1 ou 2, **caractérisée en ce que** le collagène d'éponge marin est sélectionné parmi des éponges marines de la classe des Demospongiae et de ses 11 ordres Geodiidae, Chondrosiidae, Suberitidae, Axinellidae, Halichondriidae, Dysideidae, Spongiidae ou des mélanges de celles-ci.

4. Préparation selon l'une des revendications 1 à 3, **caractérisée en ce que** l'agent d'enrobage contenant le collagène d'éponge ne contient aucun autre agent de retard résistant au suc gastrique (sélectionné par exemple parmi des (poly)acrylates, des dérivés de la cellulose, des alcools polyvinyliques ou des dérivés de ceux-ci).

5. Préparation selon l'une des revendications 1 à 4, **caractérisée en ce que** la matrice ou une forme posologique contenant celle-ci se présente sous la forme d'un comprimé, d'un granulé, d'une capsule ou d'une dragée et au moins une partie de l'excipient est du collagène d'éponge marin dénaturé.

6. Préparation selon l'une des revendications 1 à 5, **caractérisée en ce que** du collagène d'éponge natif est présent sur la matrice en tant qu'enrobage dans une quantité de 0,5 à 9 % en poids, rapporté au poids total de la préparation.

7. Préparation selon l'une des revendications 1 à 6, **caractérisée en ce que** le principe actif est sélectionné parmi la lécithine d'oeuf, de soja, l'acide fumarique, le butyrate de sodium, la coenzyme Q10, le glutathion, l'acide orotique, l'arginine, la progestérone ou l'ornithine ou des combinaisons de ceux-ci.

8. Préparation selon l'une des revendications 6 ou 7, **caractérisée en ce que** le principe actif est l'acide fumarique, la lécithine, la coenzyme Q10 ou des combinaisons de ceux-ci.

9. Préparation selon l'une des revendications 1 à 8 pour une utilisation dans la prévention ou le traitement de la sclérose en plaques ou du psoriasis, dans laquelle de l'acide fumarique est contenu en tant que principe actif.

10. Préparation selon l'une des revendications 1 à 8 pour une utilisation dans la prévention ou le traitement de la colite ulcéreuse, dans laquelle le principe actif est la lécithine.

11. Procédé pour la réalisation d'une préparation avec libération contrôlée de principe actif dans le côlon (ciblage du côlon), **caractérisé en ce que** l'on prépare d'une manière connue en elle-même une matrice ou une forme posologique contenant celle-ci, comprenant un ou plusieurs excipients, comprenant du collagène d'éponge marin, un ou plusieurs additifs à cette fin ainsi qu'un ou plusieurs principes actifs et cette matrice se mélange avec ou enveloppe une solution ou suspension contenant de l'eau ou de l'éthanol-eau d'un collagène d'éponge marin, qui présente une valeur de pH d'environ 6,5 à 8,5, dans une quantité de 0,2 à 10 % en poids, rapporté au poids total de la préparation, et une telle préparation résistante au suc gastrique est obtenue avec un profil de libération défini dans le côlon, dans lequel la matrice comprend du collagène d'éponge marin dénaturé en tant que support ou en tant que partie de la matière de support, lequel est différent d'un point de vue structural du collagène d'enrobage.

12. Procédé selon la revendication 11, **caractérisé en ce que** le collagène d'éponge est sélectionné dans la classe des Demospongiae et la suspension d'enrobage est composée de 2 à 10 % de collagène d'éponge, et de 90 à 98 % en poids d'un mélange d'eau et d'éthanol avec de 25 à 70 % d'eau et de 75 à 30 % d'éthanol, comprenant en outre une ou plusieurs substances régulatrices de la valeur de pH sélectionnées parmi l'hydrogénophosphate de di-ammonium, le carbonate de di-ammonium, NH₄OH, une solution d'ammoniaque et l'acide phosphorique, pour l'ajustement optimal de la valeur de pH, un ou plusieurs régulateurs de viscosité, et/ou un ou plusieurs conservateurs.

13. Procédé selon la revendication 11 ou 12, **caractérisé en ce que** la matrice ou une forme posologique contenant celle-ci est un comprimé, un granulé ou une capsule et le principe actif est la lécithine, la coenzyme Q10, et/ou l'acide fumarique.

14. Procédé selon l'une des revendications 11 à 13, **caractérisé en ce que** la solution/suspension d'enrobage de collagène d'éponge est pulvérisée sur la matrice.

15. Utilisation d'une préparation selon l'une des revendications 1 à 7 pour la réalisation d'un agent pour la prévention ou le traitement de la maladie d'Alzheimer, de neuropathies, **caractérisée en ce que** la préparation comprend en tant que principe actif de l'acide fumarique, et en outre de l'acide orotique ou de la coenzyme Q10.

16. Utilisation d'une solution ou suspension aqueuse ou hydro-alcoolique exempte de talc de collagène d'éponge marin avec une valeur de pH de 6,5 à 8,5, contenant des régulateurs de valeur de pH, en particulier sélectionnés parmi un tampon phosphate, l'hydroxyde d'ammonium, des tampons carbonate d'ammonium et/ou une solution d'ammoniaque et l'acide phosphorique, en tant qu'agent d'enrobage résistant au suc gastrique externe ou intégré pour la réalisation de préparations comprenant des principes actifs pour le ciblage du côlon pour la libération in vivo contrôlée du ou des principes actifs ou pour la constitution in vivo d'une barrière physiologique dans le côlon pour empêcher le passage de germes et de toxines dans des tissus et des systèmes corporels d'un mammifère, en particulier de l'homme, dans laquelle la matrice de la préparation comprend du collagène d'éponge marin dénaturé en tant que support ou en tant que partie de la matière de support, lequel est différent d'un point de vue structural du collagène d'enrobage.
